(19) Europäisches Patentamt
European Patent Office
Office européen des brevets

(11) EP 0 580 078 B1

(12) **EUROPÄISCHE PATENTSCHRIFT**

(45) Veröffentlichungstag und Bekanntmachung des Hinweises auf die Patenterteilung:
**15.10.1997 Patentblatt 1997/42**

(51) Int Cl.$^{6}$: **C08F 8/12**

(21) Anmeldenummer: **93111375.7**

(22) Anmeldetag: **15.07.1993**

(54) **Hydrophile Zentren aufweisende Polyvinylamin-Derivate, Verfahren zu ihrer Herstellung sowie die Verwendung der Verbindungen als Arzneimittel, Wirkstoffträger und Nahrungsmittelhilfsstoff**

Hydrophilic groups containing poly(vinylamine) derivatives, process for their manufacture and use thereof as pharmaceutical compounds, substrates for active substances and foodstuff ingredients

Dérivés de polyvinylamine présentant des groupes hydrophiles, leur procédé de préparation et leur application comme médicaments, supports de matières actives et ingrédients pour produits alimentaires

(84) Benannte Vertragsstaaten:
**AT BE CH DE DK ES FR GB GR IE IT LI LU MC NL PT SE**

(30) Priorität: **22.07.1992 DE 4224108**

(43) Veröffentlichungstag der Anmeldung:
**26.01.1994 Patentblatt 1994/04**

(73) Patentinhaber: **HOECHST AKTIENGESELLSCHAFT**
**65926 Frankfurt am Main (DE)**

(72) Erfinder:
- **Ahlers, Michael, Dr.**
  **W-55122 Mainz (DE)**
- **Glombik, Heiner, Dr.**
  **D-65719 Hofheim am Taunus (DE)**
- **Grabley, Susanne, Dr.**
  **D-61462 Königstein/Ts. (DE)**
- **Granzer, Ernold, Dr. Dr.**
  **D-65779 Kelkheim (DE)**
- **Müllner, Stefan, Dr.**
  **D-65239 Hochheim (DE)**
- **Walch, Axel, Dr.**
  **D-60487 Frankfurt am Main (DE)**
- **Xu, Guan-Yu, Prof.**
  **CN-200041 Shanghai (CN)**

(56) Entgegenhaltungen:
**EP-A- 0 071 050** **EP-A- 0 262 577**
**EP-A- 0 339 371** **DE-A- 4 007 311**
**FR-A- 1 587 554** **US-A- 4 018 826**

- **CHEMICAL ABSTRACTS, vol. 112, no. 14, 2. April 1990, Columbus, Ohio, US; abstract no. 119637j, ITO, TAKESHI 'FIRE-RESISTANT CHLORO-CONTAINING POLYMERS' Seite 14 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft lösliche und unlösliche stickstoffhaltige, hydrophile Zentren aufweisende Vinylpolymere, ihre Verwendung als Gallensäure-Adsorber mit dem Ziel der Senkung des Cholesterin-Blutspiegels, ihre Verwendung als Wirkstoffträger sowie als Nahrungsmittelhilfsstoff und -zusatz und ferner ein Verfahren zur Herstellung dieser Verbindungen.

Gallensäuren haben eine wichtige physiologische Funktion bei der Fettverdauung. Als Endprodukte des Cholesterinstoffwechsels werden sie in der Leber synthetisiert, in der Gallenblase gespeichert und in den Darm abgegeben, wo sie ihre physiologische Wirkung entfalten. Der größte Teil der sezernierten Gallensäuren wird über den enterohepatischen Kreislauf wieder zurückgewonnen (ca. 20-50 g/Tag). Eine Unterbindung dieser Rückresorption vermindert den Gallensäurepool in der Leber und bewirkt so neben einer Stimulierung der Cholesterineigensynthese eine verstärkte Cholesterinaufnahme aus dem Blutkreislauf. Hierzu erhöht sich die Zahl der hepatischen LDL-Rezeptoren auf den Membranen der Leberzellen, so daß die cholesterinhaltigen LDL-Partikel beschleunigt katabolisiert werden und es zu einer Herabsetzung des Cholesterinanteils im Blut kommt.

Es ist bekannt, daß sich Gallensäuren an unlösliche, basische, vernetzte Polymere wie Polyethylenimine (vgl. z. B. EP-A-0 379 161) oder Polyvinylimidazole (vgl EP-B-0 162 388) binden lassen und daher als geeignet zur Behandlung von Erkrankungen, bei denen eine Hemmung der Gallensäurerückresorption im Darm, insbesondere im Dünndarm, wünschenswert erscheint, angesehen werden. Beispielsweise werden die chologene Diarrhoe nach Ileumresektion oder auch erhöhte Cholesterin-Blutspiegel auf diese Weise behandelt.

Für die als Lipidsenker in der Verwendung befindlichen Ionenaustauscherharze wie Colestipol und Colestyramin ist insbesondere eine sehr hohe Tagesdosis einzuhalten. Sie beträgt z.B. für Colestyramin 12 - 24 g, im Höchstfall 32 g, für Colestipol 15 - 30 g.

Diese hohe Dosierung sowie unangenehmer Geruch, Geschmack und Konsistenz erschweren die Patienten-Compliance.

Nebenwirkungen dieser Ionenaustauscherharze sind auch auf die mangelnde Selektivität (z.B. Avitaminosen) zurückzuführen. Für beide Präparate wurde eine therapeutische Bedeutung in Kombination mit anderen hypolipidämisch wirkenden Pharmaka wie Fibrate, HMG-CoA-Reduktase-Inhibitoren, Probucol (vgl. z.B. M.N. Cayen, Pharmac. Ther. 29, 187 (1985) und 8th International Symposium on Atherosclerosis, Rome, Oct. 9-13, 1988, Abstracts S. 544, 608, 710) beschrieben, wobei die erzielten Effekte auch die Therapie von schweren Hyperlipidämien ermöglichen. Deshalb erscheint es bedeutungsvoll, bei dem gegebenen Wirkprinzip geeignete Stoffe ohne die Nachteile der gegenwärtig verwendeten Präparate zu finden.

Folgende Merkmale der genannten Präparate und insbesondere von Colestipol sind als verbesserungswürdig anzusehen:

1. Die hohen Tagesdosen, die auf eine geringe Bindungsrate in isotonischer Lösung und auf teilweise Wiederfreisetzung der adsorbierten Gallensäure zurückzuführen sind.

2. Die qualitative Verschiebung der Gallensäurezusammensetzung der Galle mit abnehmender Tendenz für Chenodesoxycholsäure und die damit verbundene zunehmende Gefahr der Cholelithiasis.

3. Das Fehlen einer dämpfenden Wirkung auf den Cholesterinstoffwechsel der Darmbakterien.

4. Die zu hohe Bindungsrate von Vitaminen und Pharmaka, die Substitutionsbedarf an diesen Stoffen und Blutspiegelkontrollen eventuell erforderlich macht.

5. Nicht ausreichende Reinheit und Stabilität der Polymere (bei Colestyramin Gefahr der Abspaltung von Ammonium-Gruppen).

6. Unzureichende Patienten-Compliance aufgrund a) der "sandigen" Konsistenz (Colestyramin = Hardgel-Polymer) und b) des unangenehmen Geruchs und Geschmacks.

Variationen zu den bisher verwendeten Präparaten wie z.B. Einführung von Spacern zwischen Ammonium-Gruppen und Polymerhauptkette im Fall des Colestyramins (EP-A-0 404 062) führen zu keiner entscheidenden Verringerung der beschriebenen Nachteile.

Aufgabe der vorliegenden Erfindung war es, Verbindungen mit anderen Polymerstrukturen bereitzustellen, die Gallensäuren konzentrationsabhängig in hohem Maße binden. Darüberhinaus sollten diese Verbindungen die bestehenden Nachteile der bisher verwendeten Austauscherharze nicht bzw. nicht mehr im bekannten Maße aufweisen.

Mit den stark wasserabsorbierenden Polymeren der allgemeinen Formel I gelingt die Lösung der Aufgabe sowie

die Überwindung der beschriebenen Mängel.

Die Erfindung betrifft daher Polyvinylamine der Formel I

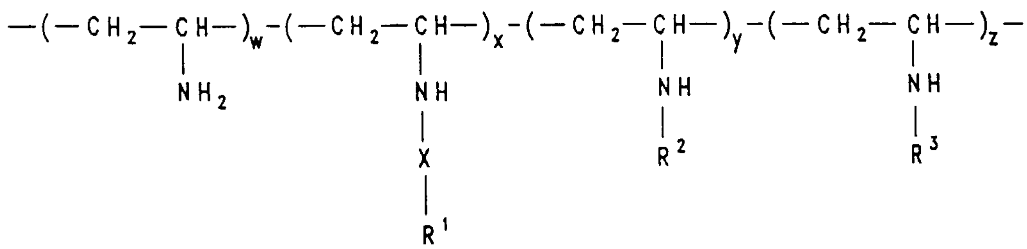

(I)

worin

$R^1$ einen Substitutenten bedeutet, ausgewählt aus der Gruppe bestehend aus:

1. $-(CH_2)_n-CH_3$, wobei n eine ganze Zahl von 3 bis 21 ist, verzweigtes Alkyl mit 3 bis 21 Kohlenstoffatomen, geradkettiges oder verzweigtes Alkenyl mit bis zu 21 Kohlenstoffatomen,
2. Cycloalkyl, Cycloalkenyl mit jeweils 5-12 Kohlenstoffatomen, ein-, zwei- oder dreifach substituiertes Cycloalkyl oder Cycloalkenyl mit jeweils 5-12 Ringkohlenstoffatomen und
3. Aryl, Arylalkyl, Arylalkenyl, wobei die Arylreste ein- oder mehrkernig sind, ein- bis dreifach substituiert sein können und Heteroatome enthalten können,

X eine Einfachbindung, eine Brückengruppe oder ein hydrophiler Spacer zur Kopplung des Restes $R^1$ ist,

$R^2$ $R_A$-Y, $R_B$ oder $R_C$ ist, wobei Y eine Brückengruppe oder ein Spacer ist, der es erlaubt, $R_A$ an das Polymer zu koppeln,

$R_A$ einen hydrophilen bzw. amphiphilen Substituenten bedeutet ausgewählt aus der Gruppe bestehend aus:

1.

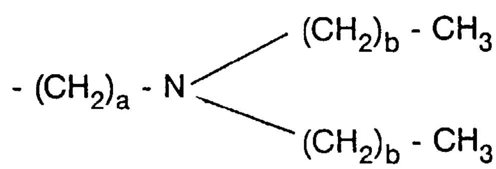

2.

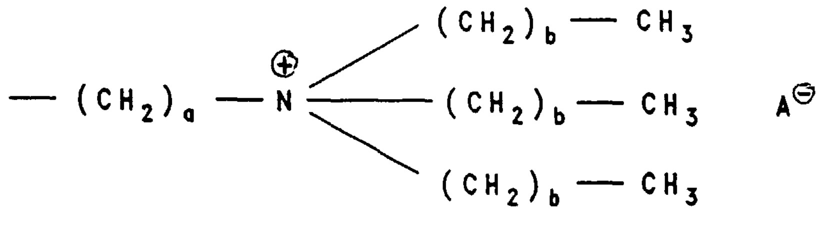

3.

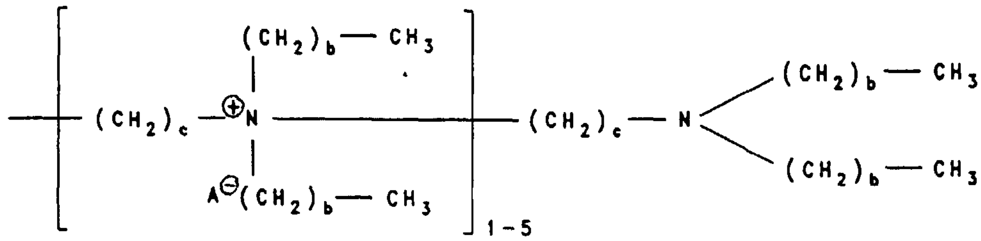

4. $-(CH_2)_c-B$, wobei B einen über N gebundenen Pyrrolidinyl-, Piperidinyl- oder Morpholinyl-Rest bedeutet,

5. $-(CH_2)_a-D^{\oplus}A^{\ominus}$, wobei $D^{\oplus}$ Pyridinium, Pyrimidinium oder Imidazolinium bedeutet,

6. 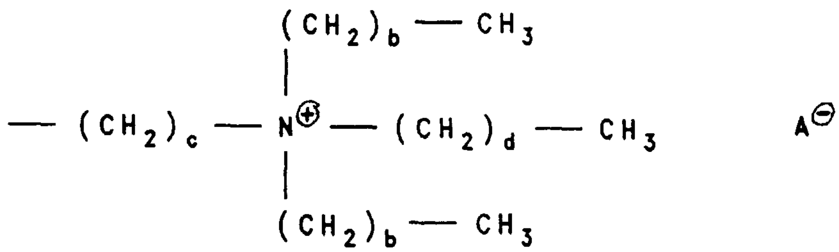

wobei bei den unter 1. bis 6. angegebenen Substituenten

a eine ganze Zahl von 2 bis 16,
b null, 1, 2 oder 3,
c eine ganze Zahl von 2 bis 6,
d eine ganze Zahl von 6 bis 17 und
A ein physiologisch verträgliches Anion bedeuten,

$R_B$ 1. eine Cholsäure, die über die 3-α-OH- oder 24-COOH-Funktion direkt oder über einen Spacer gebunden ist, darstellt, oder
2. eine Tauro- oder Glycocholsäure, die über die 3α-OH- oder Tauro- bzw. Glyco-Funktion direkt oder über einen Spacer gebunden ist,

$R_C$ ein hydrophiler cyclischer Rest oder ein Glucopyranuronsäure-Rest ist, $R^3$ einen Vernetzer bedeutet, ausgewählt aus der Gruppe bestehend aus:

1. $-(CH_2)_e-$,

2.

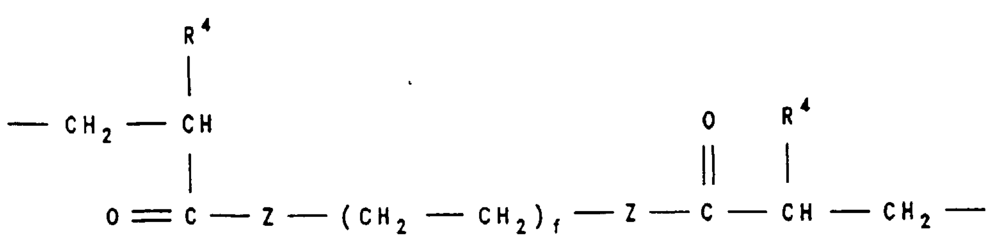

wobei Z Sauerstoff oder NH bedeutet,

3.

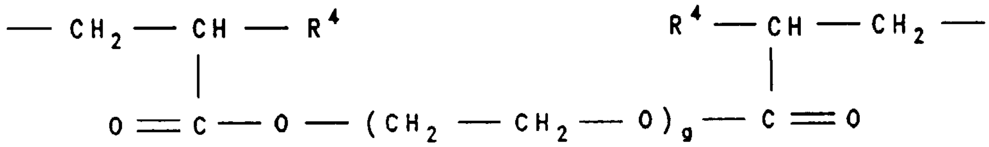

4. $-(CH_2-CH_2-O)_h-CH_2-CH_2-$,

5. $W-(CH_2)_g-W$,

wobei W eine

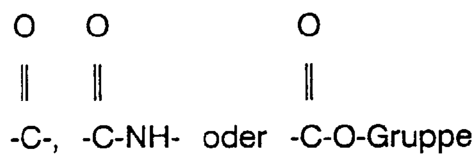

oder

ist,

6.

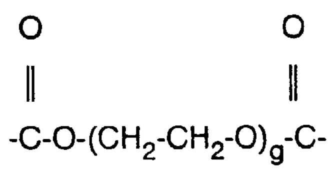

und

7.

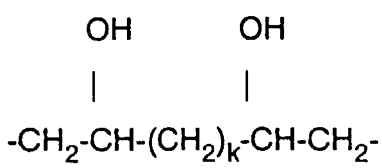

wobei bei den unter 1. bis 7. angegebenen Gruppen

- e eine ganze Zahl von 3 bis 12,
- f eine ganze Zahl von 1 bis 6,
- g eine ganze Zahl von 1 bis 8,
- h eine ganze Zahl von 1 bis 7,
- k eine ganze Zahl von 4 bis 8,
- $R^4$ Wasserstoff oder $CH_3$ bedeuten,
  und worin
- w mindestens 0,1 ist,
- x 0,0 - 0,8 ist,
- y 0,01 - 0,8 ist, und
- z null oder 0,005 - 0,3 ist, wobei w+x+y+z = 1 ist, und deren physiologisch verträgliche Salze.

Verbindungen der Formel I mit z gleich null sind unvernetzt und löslich, während die Verbindungen mit z gleich 0,005 bis 0,3 vernetzt und unlöslich sind.

In den vorstehenden und folgenden Ausführungen steht:

Aryl für einen ein- oder mehrkernigen aromatischen Kohlenwasserstoffrest mit 6 bis 14 C-Atomen, wobei im Falle der mehrkernigen Reste die Arylgruppen miteinander kondensiert, über C-C-Bindungen oder über Brückenglieder wie -O-, -COO- oder -CONH- miteinander verbunden sind.

Ferner umfaßt der Begriff Aryl auch 5- bis 14-gliedriges Heteroaryl mit 1 Heteroatom oder 2 nicht benachbarten, gleichen oder verschiedenen Heteroatomen, ausgewählt aus der Gruppe bestehend aus Sauerstoff und Stickstoff.

Aryl steht insbesondere für Phenyl, Arylalkyl für Benzyl und Phenylethyl und Arylalkenyl für

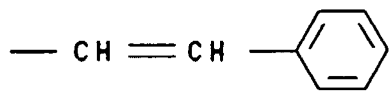 .

Beispiele für aromatische Reste mit 1 oder 2 Heteroatomen sind Reste der Chinolincarbon-, Benzimidazolcarbon-, Furancarbon-, Nicotin- und Cumarilsäure.

Die Cycloalkyl bzw. Cycloalkenylreste sind gegebenenfalls ein, zwei oder dreifach substituiert mit Hydroxy, $(C_1$-$C_6)$-Alkyl und/oder $(C_1$-$C_6)$-Alkoxy-Resten, wobei im Falle der Mehrfachsubstitution die Substituenten gleich oder verschieden sind. Entsprechendes gilt auch für die Substituenten am Aryl; als Rest kommt z.B. ein Triethylbenzoesäurerest in Frage.

Das Brückenglied X bedeutet

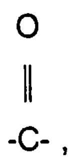 , 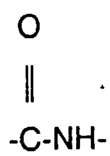 oder 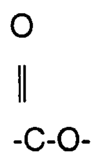 .

Der hydrophile Spacer X ist ein Rest der Formel

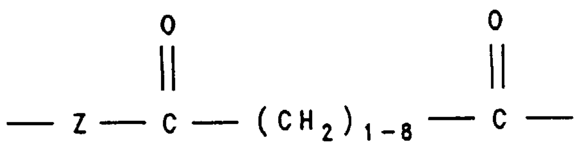

mit Z gleich Sauerstoff oder NH und wobei im Fall von 3 - 8 Methylengruppen eine mittelere $CH_2$Gruppe durch Sauerstoff ersetzt sein kann, und wobei die Alkylenkette 1 bis 4mal mit Hydroxy substituiert sein kann, oder ein Rest der Formel

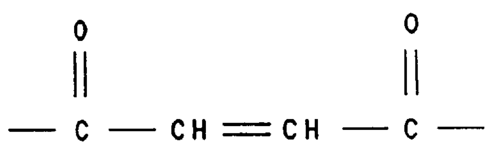

oder

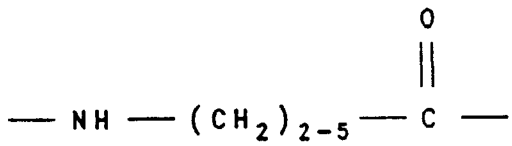

$R^1$-X ist z.B. ein Rest der Formel

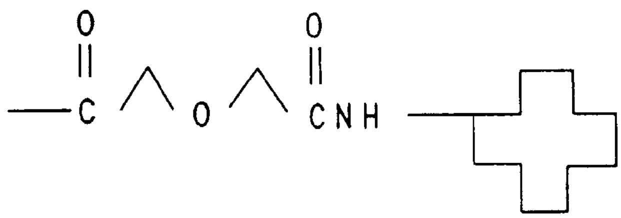

Das Brückenglied Y ist

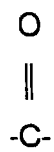 , 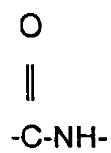 oder 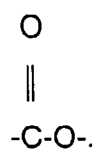.

Der Spacer Y ist ein Rest der Formel

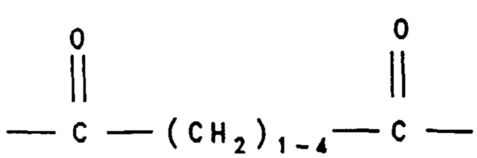 ,

wobei im Fall von 3-4 Methylengruppen eine mittlere $CH_2$-Gruppe durch Sauerstoff ersetzt sein kann.

Ein Beispiel für $R^2$ gleich $R_B$ ist der Rest der Formel

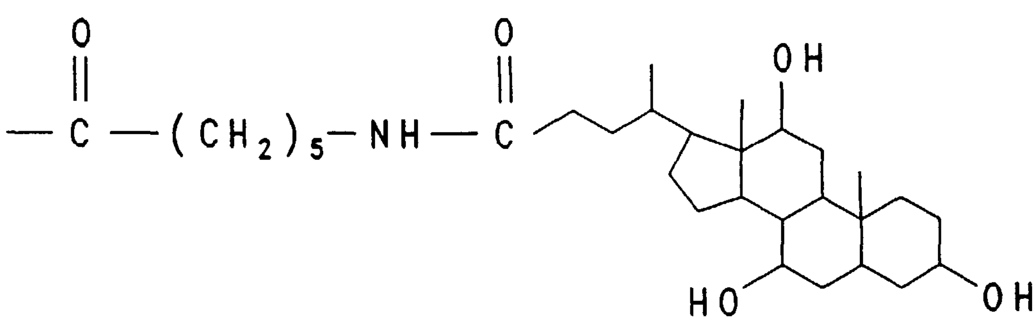

Ein hydrophiler cyclischer Rest $R_c$ ist ein Cyclodextrinrest oder ein funktionalisierter 7 - 18gliedriger C-haltiger Azamakrozyklus mit 2 bis 4 Stickstoffatomen, und gegebenenfalls 2, 3 oder 4 Sauerstoffatomen, die durch Ethylengruppen getrennt sind, wie z.B. 1,4,7-Triazacyclononan, ein Cyclen- oder Cyclamrest oder 1,4-Diaza-18 crown 6.

Die Polyvinylamine der Formel I mit z gleich null sind linear.

Wie in der Polymerchemie üblich sind die in Formel I angegebenen w, x, y und z-mal vorkommenden Molekülteile über das Gesamtpolymer statistisch verteilt oder können aufgrund von Nachbargruppeneffekten insbesondere bei hydrophoben Substituenten in Blöcken konzentriert sein.

Der Rest $R^1$ ist vorzugsweise hydrophob.

Sofern b in einer Struktur mehrfach vorkommt, so ist b gleich oder verschieden. c, $R^4$ bzw. W sind in einer Struktur immer gleich. Unter hochreinem Polyvinylamin (PVAm) bzw. PVAm-Salz werden Polymere mit einem Molekulargewicht von 10.000 bis 1.000.000 D, die nachweislich kein Restmonomer, keine freien Initiatorrestbestandteile und keine Cokomponenten im Polymer enthalten, verstanden.

Vinylamin-Polymere und deren Herstellung sind schon beschrieben worden.

Vernetztes PVAm, hergestellt aus N-Vinylcarbamidsäureisopropylester, wurde beschrieben als Anionenaustauscher (Storck, W. und Manecke, G., Makromol. Chem. 110, 207 (1967)).

In der US Patentschrift 4,018,826 wird die Herstellung von Polyvinylamin(PVAm) aus Polyvinylacetonitril und in der US Patentschrift 4,943,676 wird die partielle Thermolyse von Polyvinylformamid zu Polyvinylamin beschrieben. Copolymere aus Vinylformamid und Vinylamin, hergestellt durch Polymerisation von Vinylformamid und anschließende partielle Hydrolyse, werden beschrieben in EP-B-0 071 050 und DE-A-40 07 310.

In EP-A-0 262 577 wird ein Homopolymer mit min. $10^{+6}$ D MW (Molekulargewicht), das "hauptsächlich" aus Polyvinylamin-Einheiten besteht und durch inverse Emulsionspolymerisation hergestellt wurde, beschrieben.

EP-A-0 374 646 betrifft die Herstellung von Wasser-in-Öl-Emulsionen aus Polyvinylamin.

Entsprechend den Angaben in den vorstehend aufgeführten Publikationen eignen sich die Polyvinylamine für technische Anwendungen im nicht medizinischen Bereich, z.B. als Flockungsmittel bei der Papierherstellung, Verdicker bei der tert. Erdölförderung, Additiv für Motoröle und als Filtermembrane.

In der spanischen Patentschrift Nr. 2 006 782 wird die Herstellung eines speziellen Ionenaustauschers aus Vinylamin, Epichlorhydrin und Chlorammoniumglycidin beschrieben. Dieser Ionenaustauscher soll cholesterinsenkende Eigenschaften besitzen. Angaben über die pharmakologische Wirkung fehlen. In Kenntnis der Veröffentlichungen T. A. Augurt in Encyclopedia of Polymer Science and Technology, Vol. 14; Wiley & Sons, NY, 1971, S. 251; P. Ferruti et al., Adv. Polym. Sci, 58, 55 - 92 (1984), Bayer E. et al. Makromol. Chem. 181, 585 (1980) war die Synthese allerdings nicht nachvollziehbar.

Die Dissertation Thomas Fischer (Marburg 1992) betrifft Gallensäureadsorber auf Basis von aliphatischen Polyaminen. Die beschriebenen Polyvinylamine sind frei von zusätzlichen hydrophilen Zentren.

Schließlich werden in der US Patentschrift 4,362,711 Vesikel aus einer Polymermatrix gefüllt mit einer Lösung, die Polyvinylaminhydrochlorid als Bestandteil enthalten kann, als Gallensäuresequestrant ohne Angaben zur Wirksamkeit, beschrieben.

Es wurde nun gefunden, daß besonders die Einführung von zusätzlichen hydrophilen Zentren zu gut wirksamen Verbindungen führt.

Aufgrund des bekannten Standes der Technik wird PVAm durch Polymerisation von Vinylformamid mit anschließender Hydrolyse zu Polyvinylamin und gegebenenfalls polymeranaloger Umsetzung erhalten. Zur Vermeidung von Unverträglichkeiten aufgrund von eventuell toxischen niedermolekularen Bestandteilen wie Initiator- und Monomerreste, Antioxidatien, Regulatoren und Nebenprodukten ist es für die Anwendung auf dem Arzneimittelsektor erforderlich, diese restlos aus dem Polymer zu entfernen, was u.U. sehr aufwendig ist oder praktisch nicht durchgeführt werden kann.

Die Erfindung betrifft ferner ein Verfahren zur Herstellung von Polyvinylaminen der Formel I, dadurch gekennzeichnet, daß man in Polyvinylamin die funktionellen Gruppen $R^1$-X, $R^2$ und/oder $R^3$ nach in der Polymerchemie üblichen Methoden einführt.

Bei dem vorstehenden Verfahren wird vorzugsweise hochreines PVAm eingesetzt. Verbindungen der Formel I enthalten wahlweise oder in Kombination folgende Strukturelemente: Polymerhauptkette, hydrophile, kationische, amphiphile und hydrophobe Substituenten sowie Vernetzer. Die Verbindungen werden synthetisiert durch polymeranaloge Reaktionen. Hierzu wird das Hydrochloridsalz oder die freie Basenform des Polymers alkyliert, acyliert, durch Addition vom Michael-Typ oder an Isocyanaten substituiert oder mit Epoxiden umgesetzt.

In Wasser oder im Gemisch mit einem wassermischbaren organischen Lösungsmittel wie Dioxan, DMF, Formamid, u.a. in homogener Phase oder als Grenzphasenreaktion mit Phasenvermittler wie Natriumdodecylsulfat oder Cetyltrimethylammoniumbromid wird mit oder ohne Zusatz von Hilfsbasen wie NaOH, KOH, Triethylamin, Pyridin nach üblichen Methoden PVAm partiell alkyliert mit Mitteln der Formel R-M, wobei M Chlor, Brom, Jod, $CH_3$-$SO_2$-O oder Tosyl bedeutet und R so gestaltet ist, daß Polymere entstehen, wie in Formel I angegeben. Analog kann PVAm durch Acylierung mit den entsprechenden Säurechloriden, -bromiden oder -anhydriden umgesetzt werden. In Methanol gelingt die Acylierung mit Aktivestern wie para-Nitrophenyl-carbonsäureestern sehr gut.

Zur Herstellung von Polyvinylamin-Derivaten mit aromatischen Substituenten können Verbindungen mit geeigneten funktionellen Gruppen verwendet werden, wie z.B. Benzylchlorid, Bromethylbenzol, Zimt- und Hydroxyzimtsäure, Naphthylessigsäure, N-(ω-Bromhexyl)-carbazol, Furancarbonsäure und Nicotinsäure.

Zur Einführung von Substituenten $R^1$, die bevorzugt hydrophoben Charakter besitzen, durch Alkylierung werden beispielsweise mittel- bis langkettige n-Alkyl-, verzweigte und cyclische Alkyl- und Alkenyl-Halogenide, Mesylate und Tosylate eingesetzt. Bevorzugt sind Butyl-, Hexyl-, Dodecyl- und Hexadecylbromide. Für die Acylierung werden Hexanoyl-, Decanoyl-, Laurinsäure- und Stearinsäurechlorid verwendet. Der hydrophobe Rest $R^1$ kann durch hydrophile Spacer X - bevorzugt durch Verwendung von Bernsteinsäure- oder Diglycolsäureanhydrid - von der Polymerhauptkette entkoppelt werden.

Zur Einführung von hydrophilen Substituenten $R^2$ werden bevorzugt Halogenide von Alkyl- oder Hydroxyalkylaminen und entsprechende Ammoniumsalze wie z.B. die Hydrochloride von Dimethylaminoethylchlorid, Dimethylaminopropylchlorid, Diethylamino-ethyl-, -propyl- und -hexylchlorid sowie Brompropylpyridiniumchlorid verwendet.
Sofern in $R^2$ ein Anion $A^{\ominus}$ vorkommt, so ist dieses ein physiologisch verträgliches Anion wie $Cl^-$, $Br^-$, $HCO_3$, Malonat, Citrat, Ascorbat usw., vorzugsweise $Cl^-$,

Zur Einführung von amphiphilen Substituenten $R^2$ werden vorzugsweise ω-Bromdodecyltriethylammoniumchlorid und ω-Mesylethyldimethyldodecylammoniumchlorid verwendet, so daß wahlweise entweder das Ammoniumzentrum oder der hydrophobe Alkylteil direkt an die Polymerhauptkette gekoppelt ist. Zur Ausnutzung eines Templat-Effektes werden Gallensäuren direkt oder über einen Spacer an PVAm-Derivate gekoppelt.

Die Substitution für die polymeranalog hergestellten Derivate erfolgt mit bis zu 80 %, vorzugsweise 5-40 %, pro Rest $R^1$ bzw. $R^1X$ und für $R^2$ mit 1 - 80 %, vorzugsweise 5 - 40 %, insgesamt aber nicht mehr als 90 %, so daß mindestens 10 % der Aminogruppen des PVAm frei bzw. teils als physiologisch verträgliches Salz vorliegen.

Als Vernetzer werden sowohl hydrophile als auch hydrophobe Linker eingesetzt wie z.B. Dibromhexan, Dibrompropan, Diepoxypropylether, Epichlorhydrin, Adipinsäuredichlorid, Triethylenglykolditosylat, Ethyldiacrylamid.

Der Vernetzungsgrad variiert von 0,5 - 30 %, vorzugsweise von 3 - 15 %. Durch das Ausmaß der Vernetzung kann der Quellungsgrad von 2 ml/g bis 1 l/g in Wasser eingestellt werden. Ein Quellungsgrad von 10 - 300 ml/g, insbesondere von 50 - 200 ml/g, ist besonders bevorzugt.

Die Aufarbeitung der Softgel-Polymere erfolgt durch direkte und inverse Fällung mit einem Fällungsmittel vorzugsweise Aceton sowie durch Ultrafiltration und Gefriertrocknung.

Bei der Herstellung der funktionellen Polymere der Formel I wird von dem Grundpolymer PVAm ausgegangen, wobei es erforderlich ist, daß dieses Grundpolymer die Voraussetzungen für eine medizinische Anwendung erfüllt. Daher wird PVAm hergestellt, das es erlaubt, ein Homopolyvinylamin zu erhalten, das keine weiteren Cokomponenten im Polymer enthält und frei von niedermolekularen Verunreinigungen ist.

Dazu wird Vinylformamid z.B. in 14%iger wäßriger Lösung mit 0,5 Mol.-% Radikalstarter 4,4'-Azocyanopentansäure (ACPS) 8 Stunden bei 70°C polymerisiert. Mit 99,8 % Umsatz erhält man ein Polyvinylformamid mit einer Viskosität von 1,74 dl/g. Dieses Polymer wird durch Ultrafiltration (Membran 10.000 D) so gereinigt, daß Restmonomer oder freie Initiatorbestandteile nicht mehr nachweisbar sind (kleiner 1 ppb in 1 %iger Lsg.).
Bedingungen: Membrankassetten mit Ausschlußgrenze 10.000 D MW Minisette von Filtron, z.B. 1mal 3x10L $H_2O$/100 g Polymer und 1mal 3x10L $H_2O$/20 g Polymer.

Die benötigten Wassermengen können gereinigt, eventuell durch Aktivkohle, mehrfach verwendet werden.

Bestimmung von Vinylformamid mittels HPLC:

| | |
|---|---|
| Säule | ®LiChrosorb Si 60 (5 µm) |
| Flußrate | 0,55 ml/min |
| Druck | 360 psi |
| Detektor | UV, 225 nm |
| Retentionszeit | 7,2 min |

Vor der Bestimmung von Vinylformamid im Polyvinylformamid wird die Polymerlösung über eine gereinigte Vorsäule (Kieselgel 60) gegeben. Eichlösungen werden identisch mit der Polymerlösung behandelt.

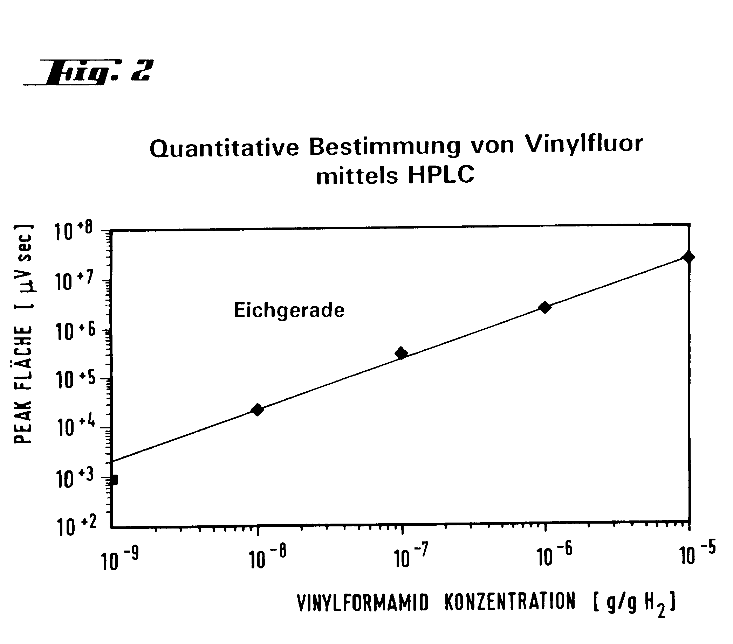

HPLC-Elutionsdiagramm siehe Figur 1 (PVAm gemäß Beispiel 1).
Eichung für den HPLC-Nachweis von Vinylformamid siehe Figur 2.

Mit 0.25 Mol.-% ACPS erhält man ein Polymer der Viskosität 2,48 dl/g, während durch Fällungspolymerisation in Isopropanol eine Viskosität von 0,31 dl/g erreicht wird. Molekulargewichte lassen sich durch die übliche Methode (Wahl der Parameter Initiator-, Monomerkonzentration und Temperatur) einstellen.

Polyvinylformamid mit sehr hohem Molekulargewicht (>1.000.000 D) wurde hergestellt, hydrolysiert zu PVAm, das aber bezüglich der Gallensäure-Adsorption geringere Wirkung als Derivate mit niederem Molekulargewicht (z.B. 75.000 D) zeigt. Es wurde gefunden, daß bei reinem PVAm, PVAm-Derivaten und deren vernetzten Folgeprodukten jeweils die Präparate mit niedrigerem MW des Ausganspolymers bessere Gallensäure-Adsorptionswerte aufweisen, als jene

mit hohem Molekulargewicht. Daher sind die Verbindungen hergestellt aus einem Ausgangspolymer mit nicht zu hohem Molekulargewicht (kleiner 1.000.000, z.B. von 10.000 bis 500.000 D) bevorzugt.

Zur Herstellung von cokomponentenfreiem PVAm wird die wäßrige Polyvinylformamid-Lösung mit 1/3 Volumen (v/v) einer starken Säure, z.B. konz. HCl, versetzt und 2 Stunden unter Rückfluß erhitzt. Danach werden ca. 2/3 des Volumens (v/v) an Säure während 6 Stunden unter Erwärmung so zugegeben, daß das Polymer gelöst bleibt. Erst nach der Hydrolyse fällt beim Abkühlen PVAm aus, so daß es von der Reaktionslösung separiert werden kann. Ameisensäure und der Überschuß an Hydrochlorid werden durch Ultrafiltration entfernt.

Eine weitere Möglichkeit der Herstellung von unlöslichen PVAm-Formulierungen ist die ionische Komplexierung der aminohaltigen Polymere mit Di-, Tri-, Tetrasäuren, Oligo- und Polysäuren zu Polyelektrolytkomplexen (PEC). Dazu wird üblicherweise eine verdünnte Lösung der Säure vorgelegt und die Lösung der Polybase so zugetropft, daß sich feine Geltröpfchen bilden, die absepariert werden können.

Die Erfindung betrifft ferner Gallensäure-Adsorbentien der Formel I, die sich gegebenenfalls in Form der Salze, insbesondere zur Behandlung von Hyperlipidämien eignen, und die Herstellung solcher Arzneimittel.

Zur Herstellung von hochwirksamen Gallensäure-Adsorbentien wurden Verbindungen der allgemeinen Formel I synthetisiert, die verglichen mit den z.Z. verwendeten Adsorbentien eine höhere Bindungskapazität aufweisen dadurch, daß

a) Polymere mit Repetiereinheiten kleiner molekularer Masse,
b) effektivere Ionenaustauschergruppen und
c) Formulierungen mit großer aktiver Oberfläche

zur Anwendung kamen.

Verbesserte Selektivität kann erreicht werden durch die Ausnutzung sowohl elektrostatischer als auch hydrophober Wechselwirkungen sowie spezieller Netzwerkstrukturen.

Durch den Einsatz der Vinylpoymere kann die übliche Dosierung der bisher verwendeten Gallensäureadsorber zur Behandlung der Hypercholesterinämie erheblich gesenkt werden. Dadurch stellt sich das Problem der Dosierung und der Compliance in geringerem Maße. Zusätzlich wird die Compliance auch dadurch verbessert, daß die Verbindungen Softgel-Charakter besitzen, geschmacks- und geruchsneutral sind, so daß keine Geschmacks- und Geruchskompensatoren benötigt werden.

Ein Vorteil der Verbindungen der Formel I ist, daß man aus diesen Verbindungen sehr leicht Filmtabletten herstellen kann. Diese zeigen in vitro die gleiche Wirksamkeit wie die Verbindungen in Pulverform. Pro 250 mg Wirkstoff enthalten sie z.B. nur 40 mg pharmazeutisch übliche Hilfsmittel.

Die mit den Verbindungen zu erzielende Senkung des Serumcholesterinspiegels kann weiter verbessert werden durch gleichzeitige Verwendung von weiteren nicht systemisch wirkenden oder systemisch wirkenden Lipidsenkern (z.B. HMG-CoA-Reduktase-Inhibitoren) im Rahmen einer Kombinationstherapie.

Da die erfindungsgemäßen Verbindungen den enterohepatischen Kreislauf unterbrechen, eignen sie sich als Antidot bei oralen Vergiftungen.

Darüberhinaus können die Verbindungen der Formel I mit z = 0,005 bis 0,3 aufgrund ihres Wasseraufnahmevermögens als Sättigungsförderer eingesetzt werden.

Da die erfindungsgemäßen Verbindungen der Formel I gut quellbar sind und Säuren binden, können sie als Antacida zur Behandlung einer übermäßigen Magensäureproduktion eingesetzt werden und somit als Mittel gegen Gastritis und Ulcus ventriculi oder duodeni Verwendung finden.

Aufgrund ihrer Wechselwirkung mit Cholesterin sind die Verbindungen in der Lage, das mit der Nahrung aufgenommene Cholesterin zu adsorbieren. Der Anteil des Cholesterins der Nahrung wird damit sofort gebunden und nicht vom Körper resorbiert.

Weiterhin eignen sich die Verbindungen der Formel I auch als Nahrungsmittelhilfsstoffe. So wird beispielsweise aus Milch oder Eibestandteilen Cholesterin adsorbiert. Die daraus resultierenden Nahrungsmittel zeichnen sich durch reduzierten Cholesterinanteil aus.

Verbindungen der Formel I sind als mucoadhäsive Transportsysteme für Wirkstoffe geeignet.
Sie bilden stark hydratisierbare Polymermatrices, die Wasserstoffbrücken-bildende sowie kationische Gruppen besitzen, eine hohe Flexibilität der Polymerkette aufweisen und zusätzlich mit hydrophoben Einheiten substituiert sein können. Daher sind die Verbindungen in der Lage, die Verweildauer eines gebundenen oder adsorbierten Wirkstoffs im Magen oder Dünndarm zu verlängern. Sie werden als Wirkstoffträger an die Schleimschicht der Magen-Darm-Wand adsorbiert, wobei die positiv geladenen Gruppen der Polymere mit den negativ geladenen Gruppen der endständigen Sialinsäuren der Mucinmoleküle in Wechselwirkung treten, um so einen verzögerten Transport der Wirkstoffe durch den Magen-Darm-Trakt zu bewirken. Gleichzeitig wird durch die Art der Wechselwirkung die Wirkstoff-Resorption verbessert.

In vitro Test

In einem in vitro Test werden Bindungskapazität und Selektivität geprüft. Hierbei kommt ein Rindergalle-Assay zur Anwendung. Dazu werden 5 mg Polymerprobe in 2 ml Testlösung gelöst und 24 Stunden bei 37°C inkubiert. Die Testlösung besteht aus Rindergalle, 1:10 verdünnt mit PBS-Puffer, pH 6,5. Die Auswertung erfolgt mittels Dünnschichtchromatographie und HPLC. Als Referenz dient Cuemid. Die Ergebnisse sind in Tabelle 1 zusammengefaßt.

Tabelle 1:

| Gallensäure-Adsorption in vitro (Rindergalle-Assay) | | |
|---|---|---|
| Beispiel | Gallensäure-Adsorption (%) | |
| | Taurocholat | Glycocholat |
| 4 | 69 | 72 |
| 5 | 64 | 68 |
| 6 | 65 | 71 |
| 7 | 63 | 58 |
| 8 | 58 | 60 |
| 9a | 48 | 50 |
| b | 44 | 44 |
| c | 60 | 54 |
| d | 36 | 38 |
| e | 36 | 38 |
| f | 32 | 30 |
| 10 | 42 | 20 |
| 11 | 68 | 65 |
| 12 | 63 | 63 |
| 13 | 54 | 51 |
| Colestyramin | 38 | 24 |

In vivo Test

An cholesteringefütterten Kaninchen wurde die Wirkung von vier Präparaten auf eine Senkung des Serumcholesterinspiegels getestet.
Dazu wurde nach einer Vorfütterungsperiode (zur Anhebung des Cholesterinspiegels) der Cholesterinspiegel bestimmt (= Vorwert). Den randomisierten Versuchsgruppen à 5 Tieren wurden über 4 Wochen 2 % cholesterin-haltige Nahrung sowie die Präparate in Konzentrationen von 12,5 - 50 mg/kg (bzw. 100 - 500 mg für Colestyramin) verfüttert. Die Änderung des Serumcholesterins gegenüber dem Vorwert ist für jedes Präparat sowie für Colestyramin als Vergleichssubstanz in Tabelle 2 angegeben.

Tabelle 2:

| Änderung des Gesamtcholesterins an 0,2 % Cholesterin gefütterten Kaninchen | | |
|---|---|---|
| Präparate | Dosierung | Änderung Serumcholesterin gegenüber Vorwert |
| Kontrolle ®Tylose | 1 % | + 4 mmol/l |
| Beisp. 5 | 50 mg/kg | -2,5 mmol/l |
| Beisp. 6 | 50 mg/kg | -1 mmol/l |
| Beisp. 7 | 50 mg/kg | +0,5 mmol/l |
| Beisp. 8 | 50 mg/kg | -0,4 mmol/l |
| Colestyramin | 500 mg/kg | -0,3 mmol/l |

Beispiel 1

Vinylamin-Homopolymer für pharmazeutische Qualität
MW: - 380.000 D
1,8 l entionisiertes Wasser werden auf 60°C erwärmt, entgast und mit $N_2$ gespült.
300 g Vinylformamid, 3 ml konz. $NH_4OH$ und 6 g ACPS werden zugegeben, und das Ganze wird 8 Stunden bei 70°C gerührt. Durch $J_2$-Titration wird Monomer-Umsatz verfolgt, der nach 8 Stunden 99,8 % beträgt.
Die Lösung wird 4mal mit Wasser auf je 20 l verdünnt und je 4mal mittels Ultrafiltration (5x10 K Minisette von Filtron) auf 2,5 l aufkonzentriert und danach gefriergetrocknet. Man erhält ein rein weißes Produkt mit einem Restmonomergehalt von 1,4 ppm.

50 g des Polymers werden erneut in Wasser gelöst, auf 10 l verdünnt, 4mal ultrafiltriert und danach gefriergetrocknet.
Restmonomergehalt liegt unter Nachweisgrenze von HPLC-Methode (< 0,1 ppm) und GC-MS.
Viskosität [η] in 0,5 % NaCl: 1,74 dl/g.

100 g Polyvinylformamid werden in 800 ml $H_2O$ gelöst, mit 270 ml konz. HCl versetzt und 2 Stunden unter Rückfluß erhitzt. Es werden 270 ml konz. HCl zugegeben. Es wird 4 Stunden unter Rückfluß erhitzt und nach Zugabe von weiteren 270 ml konz. HCl, 2 Stunden bei 60°C erwärmt. Bei Raumtemperatur wird die Salzsäure abdekantiert, das Polymer in $H_2O$ gelöst und der pH-Wert mit NaOH auf 4 eingestellt. Es wird 4mal mit je 20 1 ultrafiltriert und dann gefriergetrocknet. Laut $^1$H-300MHz-NMR enthält das Polymer keine, d.h. unter der Nachweisgrenze, (<0,05 %) Formamidgruppen mehr (kein Peak zwischen 8 und 8,5 ppm).

Die freie Basenform des PVAm läßt sich aus obigem Polymer durch Verwendung von Alkalilauge und anschließende Dialyse und Gefriertrocknung oder durch Anwendung von Ionenaustauscherharzen erhalten.

Beispiel 2

Vinylamin-Homopolymer für pharmazeutische Qualität
MW: - 75.000 D
Zu 500 ml Isopropanol werden 100 g Vinylformamid, 0,5 ml konz. $NH_3$ und 1,5 g ACPS gegeben. Es wird 6 Stunden bei 65°C gerührt. Das Polymer wird abgesaugt, im Vakuum getrocknet, in Wasser gelöst und wie in Beispiel 1 beschrieben ultrafiltriert.
50 g Polyvinylformamid werden in 400 ml $H_2O$ gelöst und bei 50°C mit konz.
NaOH-Lauge (83 g) so versetzt, daß das Polymer nicht ausfällt. Es wird 7 Stunden bei 70°C gerührt. Das Polymer wird in Aceton gefällt, in Wasser gelöst und durch Ultrafiltration und Gefriertrocknung weiter gereinigt wie in Beispiel 1 beschrieben.

Beispiel 3

0,165 g PVAm (75.000 D aus Beispiel 2) werden in 3 ml Methanol gelöst und mit 0,4 g Cholsäure-w-amidocapronsäure-p-nitrophenylester in 10 ml DMSO versetzt. Es werden drei Tropfen Triethylamin zugegeben und 1 Stunde bei Raumtemperatur gerührt. Das Produkt wird in Ethylacetat gefällt, in Methanol/$H_2O$ gelöst, auf pH 4 eingestellt und erneut in Ethylacetat gefällt. Das Polymer wird abgesaugt und im Vakuum getrocknet.
Ausbeute: 310 mg
Substitutionsgrad: 12 %

Beispiel 4

2 g PVAm (Beispiel 1, bas. Form) werden in 80 ml $H_2O$ gelöst, mit 4,5 g 6-Bromhexylpyrimidiniumbromid und 560 mg NaOH versetzt und 9 h bei 90°C gerührt.
Zur Aufarbeitung wird der Ansatz mit 1N HCl angesäuert (pH 1) und invers mit Aceton gefällt. Das Produkt wird in $H_2O$ gelöst, mit NaOH auf pH 4 titriert und erneuet mit Aceton gefällt. Nach einer weiteren Fällung in Aceton wird das erhaltenes Produkt aus $H_2O$ gefriergetrocknet.
Ausbeute: 4 g

Beispiel 5

360 g PVAm x HCl (24 % Cl) werden in 5 l $H_2O$ gelöst. Nach Einstellen des pH-Wertes auf 10 werden 323 g 6-Bromhexylpyridiniumbromid und 72 g NaOH zugegeben. Es wird unter $N_2$ 10 Stunden bei 90°C gerührt. Danach wird mit Salzsäure neutralisiert und mit 30 l $H_2O$ ultrafiltriert (cut off: 10.000 D).

Substitutionsgrad laut NMR: 15,7 %.

Der Ansatz (4 l) wird mit NaOH auf pH 10 eingestellt und mit 69 g 1,6-Dibromhexan und 22 g NaOH versetzt. Unter $N_2$ und sehr schneller Rührung wird auf 90°C erwärmt. Nach ca. 1 1/2 Stunden setzt die Gelbildung ein. Für weitere 4 1/2 Stunden wird bei 90°C gerührt. Zur Aufarbeitung wird der Ansatz mit 2 l 2 N Salzsäure angesäuert und mit 10 l Aceton invers gefällt. Der Bromid/Chlorid-Austausch erfolgt durch Quellung des Polymers in 2 N Salzsäure. Danach wird erneut mit Aceton invers gefällt, das Produkt in 8 l $H_2O$ aufgenommen und der pH-Wert mit verd. Natronlauge auf 5 eingestellt. Nach Fällung in Aceton wird im Vakuum bei 50°C getrocknet.
Ausbeute: 460 g.

Beispiel 6

140 g PVAm x HCl werden in 4 l $H_2O$ gelöst und mit NaOH auf pH 11 eingestellt. Nach Zugabe von 32 g Dimethylaminoethylchlorid-Hydrochlorid und 18 g NaOH wird unter $N_2$ 9 Stunden bei 85°C gerührt.
Mit Salzsäure wird pH 1 eingestellt und der Ansatz mit Aceton gefällt. Das Produkt wird in $H_2O$ gelöst, pH 5 eingestellt, ultrafiltriert und gefriergetrocknet.
Substitutionsgrad: 6 %
Ausbeute: 150 g.

Beispiel 7

110 g PVAm aus Beispiel 1 werden in 4 l $H_2O$ gelöst und mit 36,8 g Dimethylaminoethylchlorid Hydrochlorid und 25,6 g NaOH (in 200 ml $H_2O$) versetzt. Es wird 9 Stunden bei 90°C erhitzt. Unter schnellem Rühren werden 80 g Dibromhexan zugegeben. Nach 2 Stunden geht die Lösung in ein Gel über. Die Temperatur wird für 9 Stunden bei 85 - 90°C gehalten. Der Ansatz wird mit 3 Teilen Aceton (v/v) verdünnt und das Gel extrahiert. Die Lösung wird abdekantiert und der Rest in weiterem Aceton ausgerührt. Das Produkt wird in Wasser/Ethanol aufgequollen, der pH-Wert wird zunächst auf 1 eingestellt, das Polymer mit Aceton gefällt und erneut bei pH 4 in Wasser gequollen. Nach einer weiteren Fällung mit Aceton wird schließlich im Vakuum bei 50°C getrocknet.

Beispiel 8

100 g PVAm x HCl werden in 2,5 l $H_2O$ gelöst und mit NaOH auf pH 11 eingestellt. Nach Zugabe von 87 g (3-Brompropyl)-trimethylammoniumchlorid und 27 g NaOH wird unter $N_2$ 17 Stunden unter Rückfluß gekocht. Die Aufarbeitung erfolgt analog zu Beispiel 6 durch Ansäuern, Fällung in Aceton und Ultrafiltration.
Substitutionsgrad: 20 %.
Ausbeute: 99 g.

Beispiel 9 a, b, c, d, e, f

2 g PVAm x HCl (33 mmol) werden in 70 ml $H_2O$ vorgelegt und mit 10 ml Natronlauge auf pH 10 eingestellt. In 6 Ansätzen (a - f) werden 0,26 g; 0,64 g; 1,28 g; 1,92 g; 2,56 g bzw. 3,19 g 12-Bromdodecyl-trimethylammoniumbromid mit der jeweiligen 1,1-fachen molaren Menge NaOH in 10 ml $H_2O$ gelöst und zugetropft. Die Ansätze werden 7 Stunden bei 80°C gerührt.
Die Aufarbeitung der Ansätze a, b und c erfolgt analog Beispiel 4; zur Aufarbeitung der Ansätze d, e und f werden diese gefriergetrocknet. Das Produkt wird in Methanol/$H_2O$ aufgenommen und in Aceton/Diisopropylether 3:2 gefällt. Die Fällung wird bei pH 1 und pH 4 wiederholt. Die Ansätze werden aus $H_2O$ gefriergetrocknet.

| | | |
|---|---|---|
| 9a) | Substitutionsgrad: | 2 % |
| | Ausbeute: | 58 % |
| b) | Substitutionsgrad: | 5 % |
| | Ausbeute: | 54 % |
| c) | Substitutionsgrad: | 8 % |
| | Ausbeute: | 40 % |
| d) | Substitutionsgrad: | 12 % |
| | Ausbeute: | 88 % |
| e) | Substitutionsgrad: | 19 % |
| | Ausbeute: | 85 % |
| f) | Substitutionsgrad: | 25 % |
| | Ausbeute: | 91 % |

Beispiel 10

10 ml einer wäßrigen Lösung von Polyacrylsäure (Polyscience, 450 kD) werden in einer Konzentration von 1 mg/ml vorgelegt. Mittels einer Kanüle (Durchmesser 0,6 mm) werden 10 ml der Verbindung aus Beispiel 1 in einer Konzentration von 1 mg/ml zugetropft. Es entsteht ein Gel, das gefriergetrocknet wird und laut Analyse aus 50 % Polybase des Beispiels 1 besteht.

Beispiel 11

1 g PVAm (Beispiel 1, salzfreie Form) wird in 40 ml $H_2O$ gelöst, mit 588 mg Benzylchlorid versetzt und 8 Stunden bei 90°C gerührt. Nach Zugabe von 2,25 g 6-Bromhexylpyridiniumbromid und 470 mg NaOH wird 8 Stunden bei 90°C gerührt. Die Aufarbeitung erfolgt analog zu Beispiel 4.
Substitutionsgrad: 18 % Benzyl, 28 % Hexylpyridinium
Ausbeute: 0,76 g

Beispiel 12

1 g PVAm (Beispiel 1, salzfreie Form) wird in 40 ml $H_2O$ gelöst, mit 588 mg Benzylchlorid versetzt und 8 Stunden bei 90°C gerührt. Nach Zugabe von 1,96 g 3-Brompropylpyridiniumbromid und 470 mg NaOH wird 8 Stunden bei 90°C gerührt. 23 ml der Lösung werden zur Aufarbeitung wie in Beispiel 4 beschrieben, behandelt.
Substitution: 18 % Benzyl, 8 % Propylpyridinium
Ausbeute: 0,66 g

Beispiel 13

23 ml der Reaktionslösung aus Beispiel 12 werden mit 430 mg Dibromhexan und 150 mg NaOH versetzt und 8 Stunden bei 90°C gerührt. Die Aufarbeitung erfolgt analog Beispiel 7.
Ausbeute: 0,96 g

Beispiel 14

1,35 g Trimethyldodecylammoniumchlorid-substituiertes PVAm - 0,25 (erhalten als freies Amin aus Beispiel 9f mittels Ionenaustauscher Amberlite 400) werden in 60 ml Ethanol/$H_2O$ 1:1 gelöst und mit 1,8 g Capronsäure-p-Nitrophenylester in 20 ml Ethanol versetzt. Es wird 2 Stunden bei Raumtemperatur und 1/2 Stunde bei 40°C gerührt. Der pH-Wert wird mit Salzsäure auf 6 eingestellt, Ethanol abrotiert und die wäßrige Phase gefriergetrocknet. Aus Ethanol/Isopropanol 1:1 wird das Polymer in Ether gefällt, in $H_2O$ gelöst, dialysiert (cut off: 15.000) und gefriergetrocknet. Ausbeute: 1,5 g.

## Patentansprüche

**1.** Hydrophile Zentren aufweisende Polyvinylamin-Derivate der Formel I

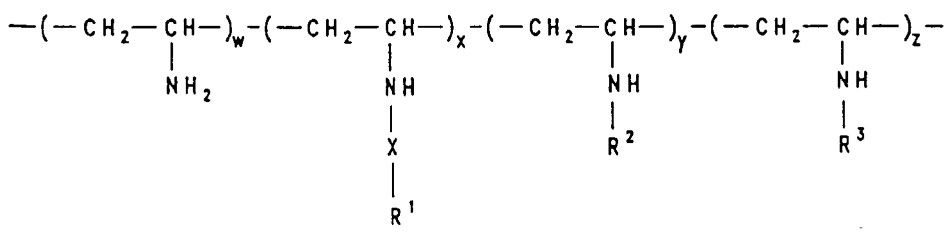

(I)

worin

$R^1$ einen Substitutenten bedeutet, ausgewählt aus der Gruppe bestehend aus:

1. $-(CH_2)_n-CH_3$, wobei n eine ganze Zahl von 3 bis 21 ist, verzweigtes Alkyl mit 3 bis 21 Kohlenstoffatomen, geradkettiges oder verzweigtes Alkenyl mit bis zu 21 Kohlenstoffatomen,
2. Cycloalkyl, Cycloalkenyl mit jeweils 5-12 Kohlenstoffatomen, ein-, zwei- oder dreifach substituiertes Cycloalkyl oder Cycloalkenyl mit jeweils 5-12 Ringkohlenstoffatomen und
3. Aryl, Arylalkyl, Arylalkenyl, wobei die Arylreste ein- oder mehrkernig sind, ein- bis dreifach substituiert sein können und Heteroatome enthalten können,

X eine Einfachbindung,

eine Brückengruppe oder
ein hydrophiler Spacer zur Kopplung des hydrophoben Restes $R^1$ ist,

$R^2$ $R_A$-Y, $R_B$ oder $R_C$ ist, wobei
Y eine Brückengruppe der Formel

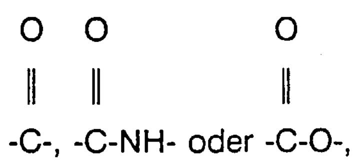

oder ein Spacer der Formel

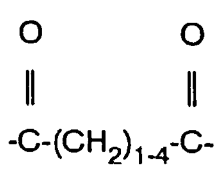

ist,
wobei im Fall von 3 - 4 Methylengruppen eine mittlere $CH_2$-Gruppe durch Sauerstoff ersetzt sein kann, der es erlaubt, $R_A$ an das Polymer zu koppeln,

$R_A$ einen hydrophilen bzw. amphiphilen Substituenten bedeutet ausgewählt aus der Gruppe bestehend aus:

1. 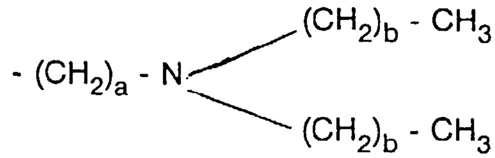

2. 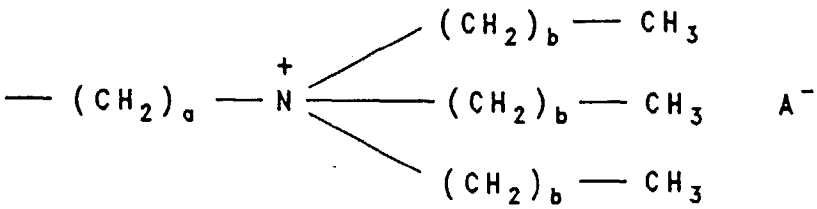

3. 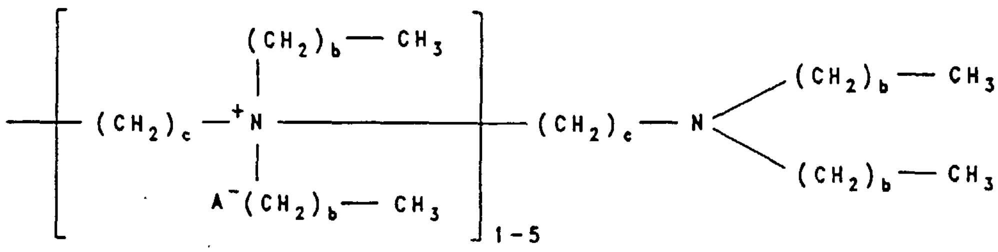

4. $-(CH_2)_c$-B, wobei B einen über N gebundenen Pyrrolidinyl-, Piperidinyl- oder Morpholinyl-Rest bedeutet,
5. $-(CH_2)_a-D^{\oplus}A^{\ominus}$, wobei $D^{\oplus}$ Pyridinium, Pyrimidinium oder Imidazolinium bedeutet,

6. 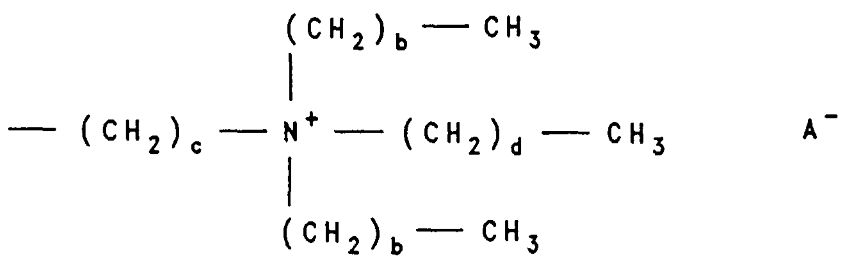

wobei bei den unter 1. bis 6. angegebenen Substituenten

a eine ganze Zahl von 2 bis 16,
b null, 1, 2 oder 3,
c eine ganze Zahl von 2 bis 6,
d eine ganze Zahl von 6 bis 17 und
A ein physiologisch verträgliches Anion bedeuten,

$R_B$ 1. eine Cholsäure, die über die 3-α-OH- oder 24-COOH-Funktion direkt oder über einen Spacer

gebunden ist, darstellt, oder
2. eine Tauro- oder Glycocholsäure, die über die 3α-OH- oder Tauro- bzw. Glyco-Funktion direkt oder über einen Spacer gebunden ist,

$R_c$ ein hydrophiler cyclischer Rest oder ein Glucopyranuronsäure-Rest ist

$R^3$ einen Vernetzer bedeutet, ausgewählt aus der Gruppe bestehend aus:

1. $-(CH_2)_e-$,

2.

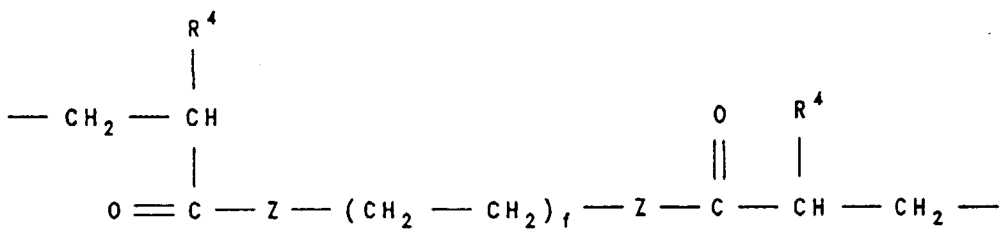

wobei Z Sauerstoff oder NH bedeutet,

3.

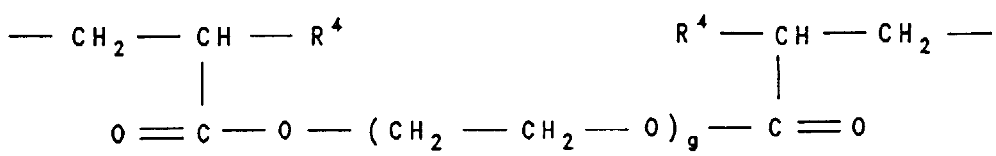

4. $-(CH_2-CH_2-O)_h-CH_2-CH_2-$,

5. $W-(CH_2)_g-W$,

wobei W eine

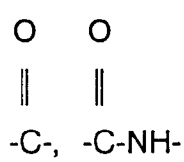 oder 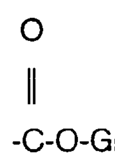Gruppe

ist,

O ‖ 

6. $-C-O-(CH_2-CH_2-O)_g-C-$ (with O above each C, joined by a double bond ‖)

und

7. $-CH_2-CH(OH)-(CH_2)_k-CH(OH)-CH_2-$ (with OH above each CH)

wobei bei den unter 1. bis 7. angegebenen Gruppen

- e eine ganze Zahl von 3 bis 12,
- f eine ganze Zahl von 1 bis 6,
- g eine ganze Zahl von 1 bis 8,
- h eine ganze Zahl von 1 bis 7,
- k eine ganze Zahl von 4 bis 8,
- $R^4$ Wasserstoff oder $CH_3$ bedeuten,
  und worin
- w mindestens 0,1 ist,
- x 0,0 - 0,8 ist,
- y 0,01 - 0,8 ist, und
- z null oder 0,005 - 0,3 ist, wobei w+x+y+z = 1 ist und deren

physiologisch verträgliche Salze.

**2.** Polyvinylamin-Derivate gemäß Anspruch 1, dadurch gekennzeichnet, daß z null ist.

**3.** Polyvinylamin-Derivate gemäß Anspruch 1, dadurch gekennzeichnet, daß z 0,005 bis 0,3 ist.

**4.** Verfahren zur Herstellung von Polyvinylaminen der Formel I und den physiologisch verträglichen Salzen gemäß Anspruch 1 oder gemäß Anspruch 2, dadurch gekennzeichnet, daß man in Polyvinylamine, die funktionellen Gruppen $R^1$-X, $R^2$ und/oder $R^3$ nach in der Polymerchemie üblichen Methoden einführt.

**5.** Polyelektrolytkomplexe erhalten aus Verbindungen gemäß Anspruch 2 und üblichen Polysäuren.

**6.** Verwendung einer Verbindung gemäß Anspruch 1 zur Herstellung eines Hypolipidämikums.

**7.** Polyvinylamin-Derivat gemäß Anspruch 1 zur Verwendung zur Adsorption von Cholesterin.

**8.** Polyvinylamin-Derivat gemäß Anspruch 1 zur Verwendung als Antidot oder Antiacidum.

**9.** Polyvinylamin-Derivat gemäß Anspruch 1 zur Verwendung in Kombination mit systemisch oder nicht systemisch wirkenden Hypolipidämika.

**10.** Polyvinylamin-Derivat gemäß Anspruch 3 zur Verwendung als Sättigungsförderer.

**11.** Polyvinylamin-Derivat gemäß der Ansprüche 2 oder 3 zur Verwendung als Wirkstoffträger.

## Claims

**1.** A polyvinylamine derivative containing hydrophilic centers, of the formula I

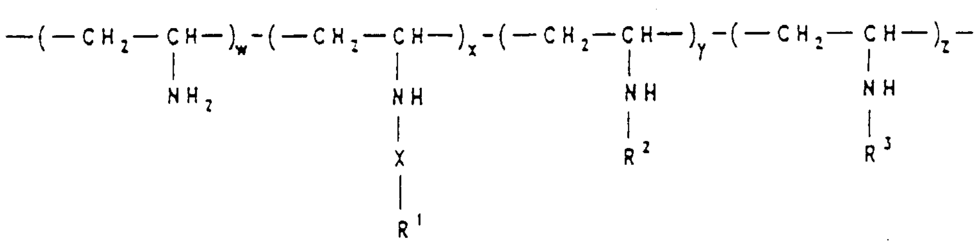

(I)

in which

$R^1$ is a substituent chosen from the group comprising:

1. $-(CH_2)_n-CH_3$, in which n is an integer from 3 to 21, branched alkyl having 3 to 21 carbon atoms or straight-chain or branched alkenyl having up to 21 carbon atoms,
2. cycloalkyl or cycloalkenyl having in each case 5-12 carbon atoms, or mono-, di- or trisubstituted cycloalkyl or cycloalkenyl having in each case 5-12 ring carbon atoms and
3. aryl, arylalkyl or arylalkenyl, in which the aryl radicals are mono- or polynuclear, can be mono- to trisubstituted and can contain heteroatoms,

X is a single bond,

a bridge group or
a hydrophilic spacer for linking the hydrophobic radical $R^1$,

$R^2$ is $R_A$-Y, $R_B$ or $R_C$, in which
Y is a bridge group of the formula

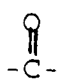
,
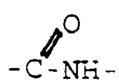
or
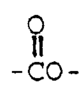

or a spacer of the formula

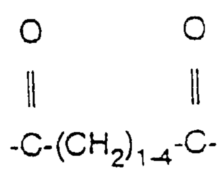

in which, in the case of 3-4 methylene groups, a central $CH_2$ group can be replaced by oxygen, which allows $R_A$ to be linked to the polymer

$R_A$ is a hydrophilic or amphiphilic substituent chosen from the group comprising:

1.

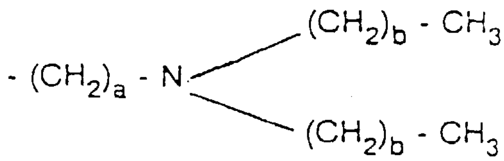

2.

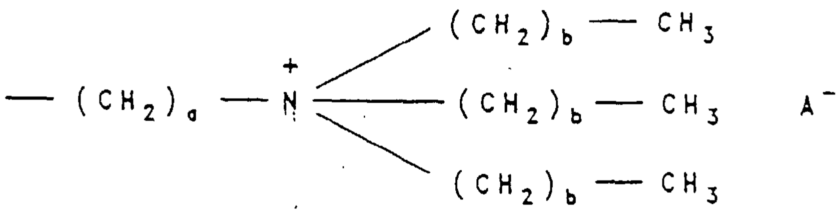

3.

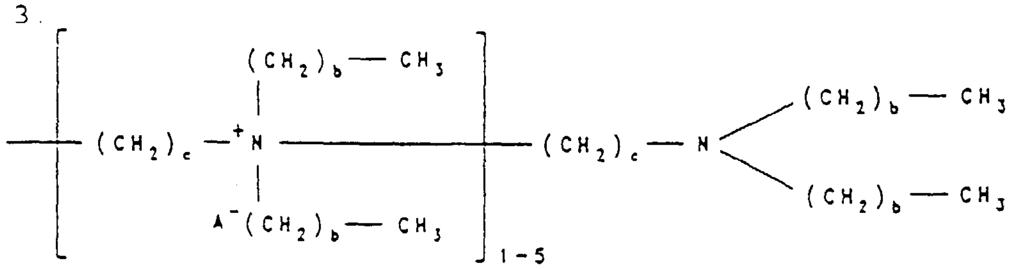

4. $-(CH_2)_c$-B, in which B is a pyrrolidinyl, piperidinyl or morpholinyl radical bonded via N,
5. $-(CH_2)_a$-$D^{\oplus}A^{\ominus}$, in which $D^{\oplus}$ is pyridinium, pyrimidinium or imidazolinium,

6.

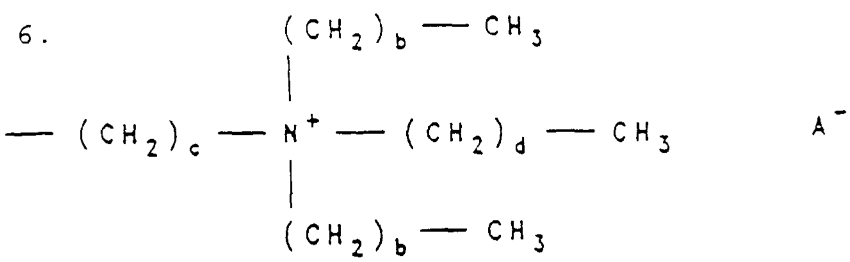

in which, in the substituents described under 1. to 6.,

a is an integer from 2 to 16,
b is zero, 1, 2 or 3,
c is an integer from 2 to 6,
d is an integer from 6 to 17 and
A is a physiologically tolerated anion,

$R_B$ 1. is a cholic acid which is bonded via the 3-α-OH or 24-COOH function directly or via a spacer, or
2. is a tauro- or glycocholic acid which is bonded via the 3α-OH or tauro or glyco function directly or via a spacer,

$R_C$ is a hydrophilic cyclic radical or a glucopyranuronic acid radical,

$R^3$ is a crosslinking group chosen from the group comprising:

1. $-(CH_2)_e-$,

2.

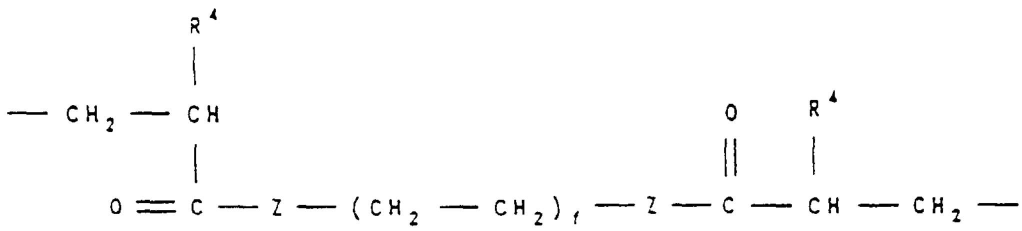

in which Z is oxygen or NH,

3.

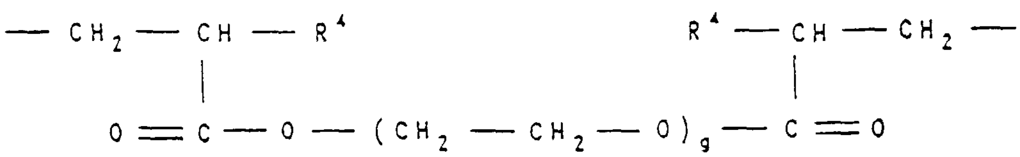

4. $-(CH_2-CH_2-O)_h-CH_2-CH_2-$,

5. $W-(CH_2)_g-W$,

in which W is a

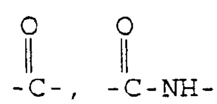

or

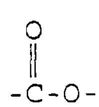

group,

6. 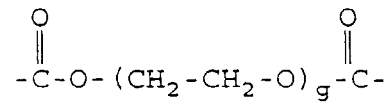

and

7. 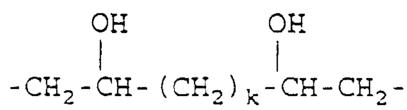

in which, in the groups described under 1. to 7.,

e is an integer from 3 to 12,
f is an integer from 1 to 6,
g is an integer from 1 to 8,
h is an integer from 1 to 7,
k is an integer from 4 to 8 and
$R^4$ is hydrogen or $CH_3$,
and in which
w is at least 0.1,
x is 0.0 - 0.8,
y is 0.01 - 0.8 and
z is zero or 0.005 - 0.3, and w+x+y+z = 1, or a physiologically tolerated salt thereof.

**2.** A polyvinylamine derivative as claimed in claim 1, in which z is zero.

**3.** A polyvinylamine derivative as claimed in claim 1, in which z is 0.005 to 0.3.

**4.** A process for the preparation of a polyvinylamine of the formula I or a physiologically tolerated salt as claimed in claim 1 or as claimed in claim 2, which comprises introducing the functional groups $R^1$-X, $R^2$ and/or $R^3$ into polyvinylamines by methods customary in polymer chemistry.

**5.** A polyelectrolyte complex obtained from a compound as claimed in claim 2 and a customary polyacid.

**6.** The use of a compound as claimed in claim 1 for the preparation of a hypolipidemic agent.

**7.** A polyvinylamine derivative as claimed in claim 1 to be used for adsorption of cholesterol.

**8.** A polyvinylamine derivative as claimed in claim 1 to be used as an antidote or antiacid.

**9.** A polyvinylamine derivative as claimed in claim 1 to be used in combination with a systemically or non-systemically acting hypolipidemic agent.

**10.** A polyvinylamine as claimed in claim 3 to be used as a satiation promoter.

**11.** A polyvinylamine derivative as claimed in claim 2 or 3 to be used as an active compound carrier.

**Revendications**

**1.** Dérivés de polyvinylamines comportant des centres hydrophiles, de formule I

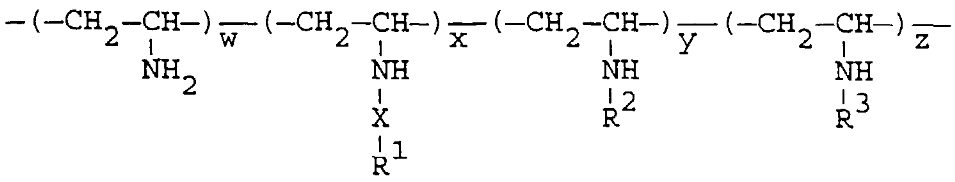 (I)

dans laquelle

$R^1$ représente un substituant choisi dans le groupe constitué par:

1. $-(CH_2)_n-CH_3$, n étant un nombre entier allant de 3 à 21,

   un groupe alkyle ramifié ayant de 3 à 21 atomes de carbone,
   un groupe alcényle à chaîne droite ou ramifié ayant jusqu'à 21 atomes de carbone,

2. un groupe cycloalkyle, cycloalcényle ayant chacun de 5 à 12 atomes de carbone, un groupe cycloalkyle ou cycloalcényle une deux ou trois fois substitué, ayant chacun de 5 à 12 atomes de carbone formant le cycle, et
3. un groupe aryle, arylalkyle, arylalcényle, les radicaux aryle étant mono- ou polynucléaires, pouvant être une à trois fois substitués et pouvant contenir des hétéroatomes,

X représente une liaison simple,

un chaînon pontant ou
un espaceur hydrophile pour l'attachement du radical $R^1$ hydrophobe,

$R^2$ est $R_A$-Y, $R_B$ ou $R_C$,
Y étant un chaînon pontant de formule

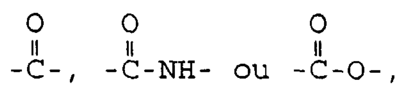
ou

ou un espaceur de formule

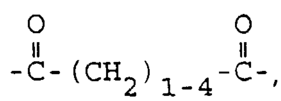

où dans le cas de 3-4 groupes méthylène, un groupe $CH_2$ central pouvant être remplacé par un atome d'oxygène,
permettant l'attachement de $R_A$ au polymère,

$R_A$ représentant un substituant hydrophile ou amphiphile, choisi dans le groupe constitué par:

1. 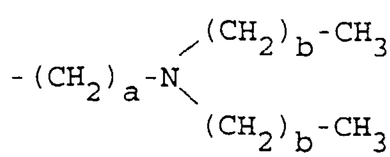

2. 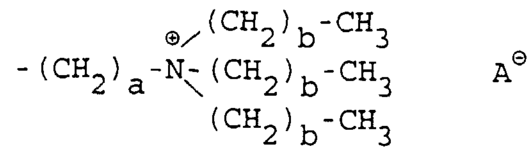

3 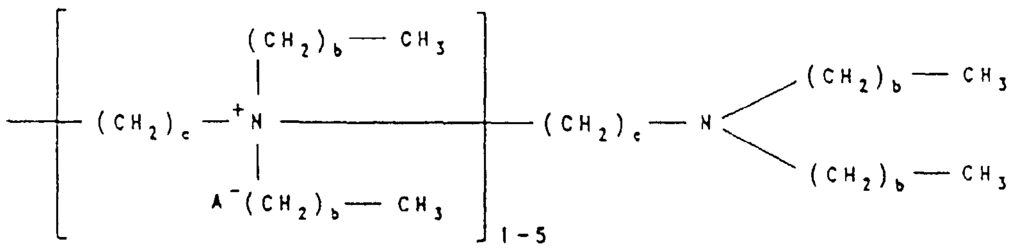

4. $-(CH_2)_c-B$, B représentant un radical pyrrolidinyle, pipéridinyle ou morpholinyle lié par N,
5. - $(CH_2)_a-D^{\oplus}$ $A^{\ominus}$, $D^{\oplus}$ représentant l'ion pyridinium, pyrimidinium ou imidazolinium,

6. 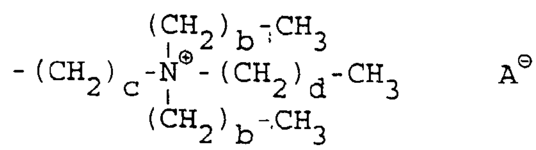

dans les substituants indiqués en 1. à 6.,

a représentant un nombre entier allant de 2 à 16,
b étant égal à 0, 1, 2 ou 3,
c représentant un nombre entier allant de 2 à 6
d représentant un nombre entier allant de 6 à 17 et
A représentant un anion physiologiquement acceptable,

$R_B$ représentant

1. un acide cholique lié par la fonction 3-α-OH ou 24-COOH, directement ou par l'intermédiaire d'un espaceur, ou
2. un acide tauro- ou glycocholique, lié par la fonction 3α-OH ou tauro ou glyco, directement ou par l'intermédiaire d'un espaceur,

$R_C$ étant un radical cyclique hydrophile ou un reste d'acide glucopyrannuronique,

$R^3$ représente un agent de réticulation, choisi dans le groupe constitué par:

1. $-(CH_2)e_4^{-}$,

2. 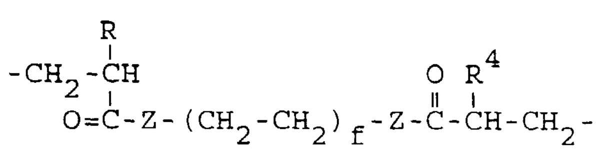

Z représentant un atome d'oxygène ou NH,

3. 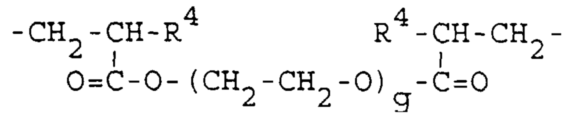

4. $-(CH_2-CH_2-O)_h-CH_2-CH_2-$,

5. $W-(CH_2)_g-W$,

W étant un groupe

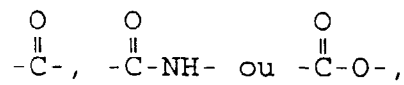

6. 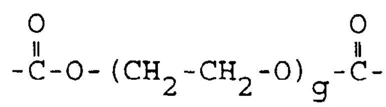

et

7. 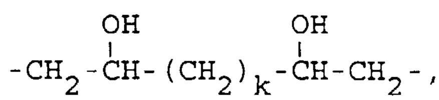

dans les groupes indiqués en 1. à 7.,

e représentant un nombre entier allant de 3 à 12,
f représentant un nombre entier allant de 1 à 6,
g représentant un nombre entier allant de 1 à 8,
h représentant un nombre entier allant de 1 à 7,
k représentant un nombre entier allant de 4 à 8,
$R^4$ représentant un atome d'hydrogène ou $CH_3$,

et dans laquelle

w est au moins 0,1,
x va de 0,0 à 0,8,
y va de 0,01 à 0,8 et
z est zéro ou 0,005-0,3, la somme w + x + y + z étant égale à 1,

et leurs sels physiologiquement acceptables.

**2.** Dérivés de polyvinylamines selon la revendication 1, caractérisés en ce que z est zéro.

**3.** Dérivés de polyvinylamines selon la revendication 1, caractérisés en ce que z va de 0,005 à 0,3.

**4.** Procédé pour la préparation de polyvinylamines de formule I et des sels physiologiquement acceptables selon la revendication 1 ou selon la revendication 2, caractérisé en ce que l'on introduit dans des polyvinylamines les groupes fonctionnels $R^1$-X, $R^2$ et/ou $R^3$, selon des méthodes utilisées habituellement dans la chimie des polymères.

**5.** Complexes de polyélectrolytes obtenus à partir de composés selon la revendication 2 et de polyacides usuels.

**6.** Utilisation d'un composé selon la revendication 1, pour la préparation d'un agent hypolipidémiant.

**7.** Dérivé de polyvinylamine selon la revendication 1, pour utilisation dans l'adsorption du cholestérol.

**8.** Dérivé de polyvinylamine selon la revendication 1, pour utilisation en tant qu'antidote ou anti-acide.

**9.** Dérivé de polyvinylamine selon la revendication 1, pour utilisation en association avec des hypolipidémiants à action systémique ou non systémique.

**10.** Dérivé de polyvinylamine selon la revendication 3, pour utilisation en tant qu'accélérateur de saturation.

**11.** Dérivé de polyvinylamine selon la revendication 2 ou 3, pour utilisation en tant que véhicule de substance active.

**Fig. 1**

## HPLC - Elutionsdiagramm

zur Bestimmung des Restmonomerengehalts in Polyvinylformamid-Lösungen

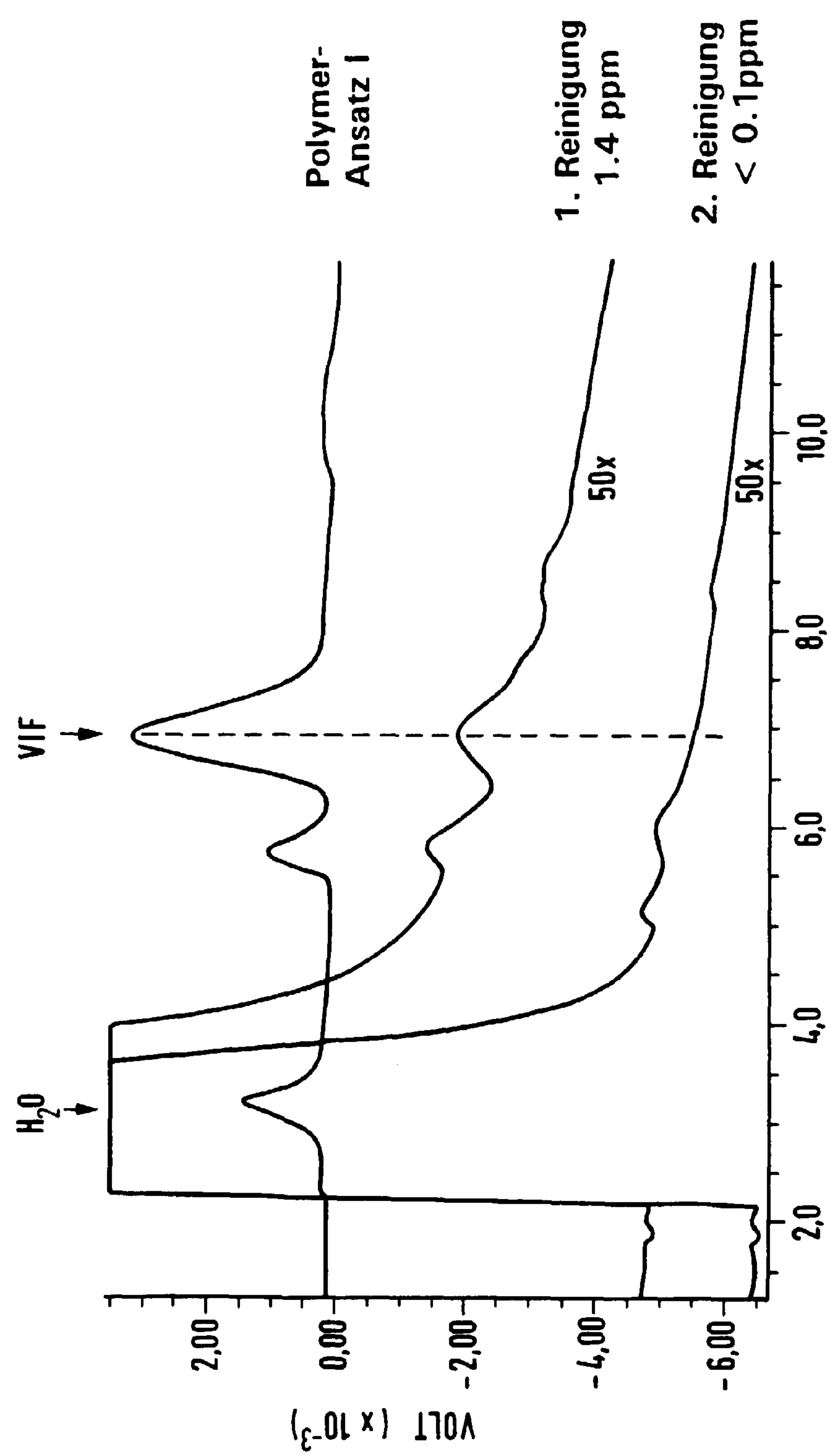

Fig. 2

Quantitative Bestimmung von Vinylfluor mittels HPLC